# EUROPEAN PATENT SPECIFICATION

(11) **EP 2 087 361 B1**
(45) Date of publication and mention of the grant of the patent: **12.01.2011**
(21) Application number: 07831767.4
(22) Date of filing: 07.11.2007
(51) Int. Cl.: G01N 33/74, C07K 14/62

(54) **METHOD FOR EVALUATING OBESITY CONTROLLER**
VERFAHREN ZUR BEWERTUNG EINER FETTLEIBIGKEIT KONTROLLIERENDEN SUBSTANZ
PROCÉDÉ POUR ÉVALUER UN RÉGULATEUR DE L'OBÉSITÉ

(30) Priority: 06.12.2006 JP 2006329637
(43) Date of publication of application: 12.08.2009
(73) Proprietor: Kao Corporation, Tokyo 1038210 (JP)
(72) Inventor: SHIMOTOYODOME, Akira, Haga-gun Tochigi 321-3497 (JP); SUZUKI, Junko, Haga-gun Tochigi 321-3497 (JP); TAKENO, Nanami, Ina-shi, Nagano 396-0021 (JP); FUKUOKA, Daisuke, Haga-gun Tochigi 321-3497 (JP); MIZUNO, Tomohito, Haga-gun Tochigi 321-3497 (JP); MEGURO, Shinichi, Haga-gun Tochigi 321-3497 (JP)
(74) Representative: Vossius & Partner
(86) International application number: PCT/JP2007/072037
(87) International publication number: WO 2008/069004

(56) References cited:
- EP-A- 1 656 838
- EP-A- 1 666 607
- WO-A-2006/109855
- ALEMZADEH R ET AL: "Antiobesity effect of diazoxide in obese zucker rats" METABOLISM, CLINICAL AND EXPERIMENTAL, W.B. SAUNDERS CO., PHILADELPHIA, PA, US, vol. 45, no. 3, March 1996 (1996-03), pages 334-341, XP004539090 ISSN: 0026-0495

## Description

### Field of the Invention

The present invention relates to a method for evaluating or screening an obesity controller, a blood insulin regulator, or a blood sugar regulator.

### Background of the Invention

Obesity is a condition in which adipose tissues have abnormally increased, and is believed to cause life-style related diseases such as diabetes, heart diseases and arteriosclerosis. In the modern society, because of high fat diet, overeating or lack of exercise, obesity is a serious problem, and search for a substance which exhibits a preventive/ameliorating effect on obesity has been an important task.

As a method for selecting an obesity ameliorating agent, there have been reported a method for measuring the amount of adiponectin expressed by mature adipocytes (Patent Document 1) ; a method for measuring the activity, or the transcription/mRNA, of UCP-2, which is one of uncoupling proteins (Patent Document 2) ; a method for measuring the level of expression or activity of SPARC gene or the SPARC protein (Patent Document 3) ; and the like. Also, as a method for selecting a weight gain suppressant, there is reported a method for evaluating a substance which stimulates the relaxin-3 receptor (Patent Document 4).

Meanwhile, insulin is a hormone secreted from pancreas as a result of stimulation mainly by an increase in the blood glucose level, that is, the blood sugar level (Non-Patent Document 1), and it is known that the amount of postprandial insulin secretion is modulated depending on the amount of carbohydrate intake or the rate of carbohydrate absorption.

However, nothing is known about the changes in the amount of insulin secretion caused by intake of both carbohydrate and lipid, and about the relationship between these changes and obesity.
[Patent Document 1] JP-A-2006-249064
[Patent Document 2] JP-A-2002-508770
[Patent Document 3] JP-A-2004-517308
[Patent Document 4] JP-A-2006-290826
[Non-Patent Document 1] Dictionary of Biochemistry, 2nd Ed., Tokyo Kagaku Dozin Co., Ltd., November 1990, pp. 141-142

### Summary of the Invention

The present invention relates to the following 1 to 6).
1) A method for evaluating or screening an obesity controller, the method including administering a test substance together with a carbohydrate and a lipid to an animal, measuring the blood insulin level by sampling the blood before administration and at time points between 1 to 300 minutes after the administration time, measuring the blood insulin level at each time point and determining an amount of secreted insulin and evaluating or selecting the substance which decreases or increases insulin secretion;
2) A method for evaluating or screening an obesity controller, the method including the following processes (1) to (4) :
   (1) a process of administering a test substance to an animal by the following methods (a) and/or (b):
      a) the test substance is added to a carbohydrate and a lipid, and/or
      b) A part or all of at least one of the carbohydrate and the lipid is replaced with the test substance;
   (2) a process of measuring a blood insulin level by sampling the blood before administration and at time points between 1 to 300 minutes after the administration time, measuring a blood insulin level at each time point and determining an amount of secreted insulin;
   (3) a process of comparing the amount of the secreted insulin determined in Process (2), with an amount of insulin secreted in a control group to which only the carbohydrate and the lipid have been administered; and
   (4) a process of evaluating or selecting, on the basis of the results of Process (3), the test substance which decreases or increases insulin secretion, as the obesity controller;
   3) A method for evaluating or screening a blood insulin regulator, the method including administering a test substance together with a carbohydrate and a lipid to an animal, measuring the blood insulin level by sampling the blood before administration and at time points between 1 to 300 minutes after the administration time, measuring the blood insulin level at each time point and determining an amount of secreted insulin and evaluating or selecting the substance which decreases or increases insulin secretion;
   4) A method for evaluating or screening a blood insulin regulator, the method including the following processes (1) to (4):
(1) a process of administering a test substance to an animal by the following methods (a) and/or (b):
   a) the test substance is added to a carbohydrate and a lipid, and /or
   b) a part or all of at least one of the carbohydrate and the lipid is replaced with the test substance;
(2) a process of measuring a blood insulin level by sampling the blood before administration and at time points between 1 to 300 minutes after the administration time, measuring a blood insulin level at each time point and determining an amount of secreted insulin;
(3) a process of comparing the amount of the secreted insulin determined in Process (2), with an amount of insulin secreted in a control group to which only the carbohydrate and the lipid have been administered; and
(4) a process of evaluating or selecting, on the basis of the results of Process (3), the test substance which decreases or increases insulin secretion, as the blood insulin regulator;
5) A method for evaluating or screening a blood sugar regulator, the method including administering a test substance together with a carbohydrate and a lipid to an animal, measuring the blood insulin level by sampling the blood before administration and at time points between 1 to 300 minutes after the administration time, measuring the blood insulin level at each time point and determining an amount of secreted insulin and evaluating or selecting a substance which decreases or increases insulin secretion; and
6) A method for evaluating or screening a blood insulin regulator, the method including the following processes (1) to (4):
   (1) a process of administering a test substance to an animal by the following methods (a) and/or (b):
      a) the test substance is added to a carbohydrate and a lipid, and/or
      b) a part or all of at least one of the carbohydrate and the lipid is replaced with the test substance;
   (2) a process of measuring a blood insulin level by sampling the blood before administration and at time points between 1 to 300 minutes after the administration time, measuring a blood insulin level at each time point and determining an amount of secreted insulin;
   (3) a process of comparing the amount of secreted insulin determined in Process (2), with an amount of insulin secreted in a control group to which only the carbohydrate and the lipid have been administered; and
   (4) A process of evaluating or selecting, on the basis of the results of Process (3), the test substance which decreases or increases insulin secretion, as the blood sugar regulator.

### Detailed Description of the Invention

The present invention relates to provide a method for efficiently evaluating or selecting an obesity controlling substance, a blood insulin regulating substance or a blood sugar regulating substance.

The present inventors conducted an investigation on postprandial insulin secretion, and as a result, found that there is an increase in the rise of a blood insulin level in the case of intake of both a carbohydrate and a lipid, compared with the case of only the carbohydrate intake. Furthermore, the inventors also conducted an investigation on the relevance between the amount of postprandial insulin secretion and obesity, and found that the rise of a blood insulin level after intake of both a carbohydrate and a lipid is highly correlated with the progress of diet-dependent obesity, and thus the rise can serve as an index for evaluating/selecting an obesity controller.

According to the present invention, a substance or composition having the effects of controlling obesity and regulating a blood insulin level and a blood sugar level can be efficiently evaluated or selected without performing a long-term feeding trial.

It is known that insulin is secreted from pancreas as a result of stimulation mainly by an increase in the blood sugar level, and the amount of secretion thereof is regulated depending on the amount of carbohydrate intake or the rate of carbohydrate absorption. However, the present invention is based on the finding that the postprandial insulin secretion (level in the blood, amount secreted) increases in the case of intake of both carbohydrate and lipid, as compared to the case of only carbohydrate intake. Furthermore, it was found that the insulin secretion after intake of both carbohydrate and lipid have high positive correlation with the increase in body weight (g) per gram of the amount of foods ingested during a feeding period, and the amount of insulin secretion is a factor highly correlated to obesity.

Therefore, the evaluation or screening of an obesity controller, a blood insulin regulator or a blood sugar regulator according to the present invention is conducted by taking as an index, the amount of insulin secretion caused by administering a carbohydrate and a lipid to an experimental animal, and specifically comprises the following processes (1) to (4):
(1) a process of administering a test substance to an animal by the following method a) and/or b):
   a) The test substance is added to a carbohydrate and a lipid, and/or
   b) A part or all of at least one of the carbohydrate and lipid is replaced with the test substance;
(2) a process of measuring a blood insulin level, by sampling the blood before administration and at time points between 1 to 300 minutes after the administration time, measuring a blood insulin level at each time point and determining an amount of secreted insulin;
(3) a process of comparing the amount of secreted insulin determined in Process (2), with an amount of insulin secreted in a control group to which only the carbohydrate and the lipid have been administered; and
(4) a process of evaluating or selecting, on the basis of the results of Process (3), the test substance which decreases or increases insulin secretion, as the obesity controller, the blood insulin regulator or the blood glucose regulator.

According to the present invention, the carbohydrate may be any carbohydrate which accelerates insulin secretion, and may be a monosaccharide, an oligosaccharide or a polysaccharide. Examples of the monosaccharide include saccharides such as glucose, mannose, fructose and galactose, or sugar derivatives such as glyceraldehyde, glucosamine and acetylglucosamine. Examples of the oligosaccharide include sucrose, maltose, lactose, and the like; and examples of the polysaccharide include starch, dextrin, glycogen and the like. These may be used singly or as a mixture of two or more thereof. Also, gelatinized rice or wheat flour containing such saccharides may also be used.
Among these, it is preferable to use one or more of glucose, sucrose, maltose, starch and glycogen, from the viewpoint of increasing the amount of secreted insulin, and glucose is more preferred.

The lipid may be exemplified by those which increase insulin secretion, for example, fatty acid glycerol esters such as triacylglycerol, 1,3-diacylglycerol and 1,2-diacylglycerol, or free fatty acids, or oils and fats containing them. Specifically, tripalmitin, tristearin, triolein, 1,3-dipalmitin, 1,3-distearin, 1,3-diolein, 1,2-dipalmitin, 1,2-distearin, 1,2-diolein, palmitic acid, stearic acid, oleic acid, beef tallow, lard, cacao butter, butter, corn oil, soybean oil, sunflower oil, sesame oil, rapeseed oil, rice oil, olive oil, safflower oil, coconut oil, palm oil and the like may be mentioned. These may be used singly or as a mixture of two or more thereof. Among these, it is preferable to use one or more of triolein, 1,3-diolein, cornoil, rapeseedoil, riceoil, olive oil, sesame oil and palm oil, from the viewpoint of increasing the amount of insulin secretion, and triolein, corn oil, rapeseed oil and olive oil are more preferred.

Such a carbohydrate and lipid may be simultaneously administered, or separately administered with a time interval. Also, an emulsified composition prepared by mixing both thereof and emulsifying them by using an emulsifier such as lecithin, may also be used.

The amount of carbohydrate to be administered is 0.01 to 100 mg/g of bodyweight, preferably 0.1 to 30 mg/g of body weight, and more preferably 0.5 to 10 mg/g of body weight.
The amount of lipid to be administered is 0.01 to 100 mg/g of body weight, preferably 0.1 to 30 mg/g of body weight, and more preferably 0.5 to 10 mg/g of body weight.

The test substance is administered together with the carbohydrate and lipid in the method of the invention. Also disclosed is that the test substance may be administered separately.

The test substance may be either the carbohydrate or the lipid, or may be a combination thereof. In the latter case, the combination effect of the test substances can be evaluated as to whether the carbohydrate or lipid to be tested induces low insulin secretion activity. Also, a test substance other than a carbohydrate or a lipid may be combined with a test substance composed of a carbohydrate or a lipid, and the combination may be subjected to evaluation.

In the case where the test substance is a carbohydrate, a part or all of the carbohydrate may be replaced with the test substance (carbohydrate to be tested) and administered together with the lipid, and in the case where the test substance is a lipid, a part or all of the lipid may be replaced with the test substance (lipid to be tested) and administered together with the carbohydrate.

The animal used for the present invention may be any kind of animal, regardless of gender and age as long as the animal is a non-humanmammal secreting insulin in the blood. For example , there may be mentioned mouse, rat, hamster, guinea pig, rabbit, cat, dog or monkey, but a rodent such as rat or mouse is preferable for being easily available and easy to handle, and it is more preferable to use mouse lines such as C57BL/6J, AKR, DBA and C3H. In addition, it is more preferable to use a fasted animal.

According to the present invention, as a method for administering carbohydrate and lipid, and a test substance to an animal, there may be mentioned, for example, oral administration, enteral administration, intraperitoneal administration, intravascular administration, intradermal administration, subcutaneous administration, and the like. However, a method of oral administration is preferred from the viewpoint of convenience or low invasiveness.

The measurement of the blood insulin level is performed by sampling the blood before administration, and at 1 to 300 minutes, preferably at 5 to 240 minutes, after the administration with time (for example, 10 minutes, 30 minutes, 60 minutes, 120 minutes, etc. after the administration), and measuring a blood insulin level at each time point. Furthermore, blood sampling may be done from general blood sampling sites such as the orbital vein or the tail vein.
Quantification of insulincanbeperformedafter preparing the serum or plasma from the sampled blood, for example, by an enzyme-linked immunosorbent assay (ELISA) method, a radioimmunoassay (RIA) method, or a high performance liquid chromatography method.

The amount of insulin secretion can be determined from the blood insulin level at each time point after the administration, using the maximum insulin level or the area under curve (AUC). Then, the changes in the values related to the insulin caused by the addition of a test substance to a carbohydrate and lipid, or by the replacement of the carbohydrate or lipid with the test substance (carbohydrate or lipid), are compared with the changes in the control group to which only the carbohydrate and lipid have been administered. When the changes become smaller in any of the cases, the test substance can be evaluated to have an effect of lowering the blood insulin level, an effect of ameliorating obesity or an effect of increasing the blood sugar level, and such a substance can be selected. Also, when one of the above-described values related to insulin increases, the test substance can be evaluated to have an effect of increasing the blood insulin level, an obesity promoting effect, an effect of lowering the blood sugar level, an effect of increasing the body weight and a growth promoting effect, and such a substance can be selected. In addition, it is believed in recent years that insulin is involved in aging, or in the changes following aging; that is, it is believed that as the amount of insulin secretion increases, aging is on the progress (Parr T. "Insulin exposure and aging theory," Gerontology. 1997; 43(3):182-200). Therefore, the evaluation method of the present invention can be used as a method for evaluating and screening an aging controller.

### EXAMPLES

### EXAMPLE 1: Increase in postprandial insulin secretion due to intake of carbohydrate and lipid

Ten fasted mice (C57BL/6J, male, 7-8 weeks old) were grouped in each group, and the mice were orally administered, using a sonde, with only 2 mg/g of body weight of glucose (Wako Pure Chemical Industries, Ltd.) as the carbohydrate, or with the carbohydrate and also 0.5, 1.0 or 2.0 mg/g of body weight of triolein (Sigma Corp.) as the lipid, in the form of an emulsion obtained by emulsifying 0.02 mg/g of body weight of the lipid with egg yolk lecithin (carbohydrate + lipid 0, carbohydrate + lipid 1, carbohydrate + lipid 2, and carbohydrate + lipid 3, respectively). The compositions of the administered products are presented in Table 1. The blood was sampled from the orbital vein of each mouse before the administration, and at 10 and 30 minutes after the administration, the blood insulin level was measured, and the area under curve (AUC) of the graph was calculated. The measurement of insulin was performed by the ELISA method (insulin measuring kit, Morinaga Biochemical Lab, Inc.).

The values of the blood insulin levels at 10 minutes after the administration, and the amount of insulin (AUC) secreted for aperiodof 30 minutes after the administration, are presented in Table 2.

**[Table 1]**

| Composition | Glucose (mg/g of body weight) | Triolein (mg/g of body weight) |
|---|---|---|
| Carbohydrate + lipid 0 | 2.0 | 0.0 |
| Carbohydrate + lipid 1 | 2.0 | 0.5 |
| Carbohydrate + lipid 2 | 2.0 | 1.0 |
| Carbohydrate + lipid 3 | 2.0 | 2.0 |

**[Table 2]**

| Postprandial insulin secretion in mouse | | |
|---|---|---|
| Administered product | Blood insulin levels at 10 minutes after administration (ng/mL) | Amount of secreted insulin for 30 minutes after administration (average AUC) |
| Carbohydrate + lipid 0 | 3.8 | 50.1 |
| Carbohydrate + lipid 1 | 5.9 | 88.0 |
| Carbohydrate + lipid 2 | 7.6 | 104.6 |
| Carbohydrate + lipid 3 | 8.1 | 111.3 |

From the results of Table 2, it can be seen that when lipid (triolein) is ingested, the postprandial insulin secretion (levels in the blood, amount secreted) increase, compared to the case of ingesting carbohydrate (glucose) alone.

### EXAMPLE 2: Relevance of postprandial insulin secretion with obesity

### (1) Measurement of postprandial insulin secretion

Triolein (Sigma Corp.) was used as the lipid, and starches (gelatinized, available from Nippon Starch Chemical Co., Ltd.) derived from sweet potato, tapioca, potato, kudzu, sago, waxy corn, sticky rice, corn, wheat and ordinary rice were used as the carbohydrate.

Four to five fasted mice (C57BL/6J, male, 10-11 weeks old) were grouped in each group, and the mice were orally administered, using a sonde, with 2 mg/g of body weight of the carbohydrate, or with the carbohydrate and also 2 mg/g of body weight of triolein plus 0.08 mg/g of body weight. The blood was sampled from the orbital vein of each mouse 10 minutes after the administration, and the blood sugar level, and the blood insulin level were measured. The blood sugar level was measured using a compact blood glucose meter (glucose dehydrogenase/potential difference measurement method, Roche Diagnostics, Inc.). The insulin level was measured by the ELISA method (insulin measuring kit, Morinaga Biochemical Lab, Inc.).

### (2) Feeding test

The testing starch used in the test diet was the above-mentioned materials, while lard, casein, cellulose, AIN76 mineral mixture, AIN76 vitamin mixture and gelatinized potato starch were obtained from Oriental Yeast Co., Ltd. , and sucrose fine granules (Special Grade) manufactured by Wako Pure Chemical Industries, Ltd. were used as sucrose. Also, the fat (TG) used was a mixture of high linoleic safflower oil, rapeseed oil and perilla oil, and the main components of fatty acid were oleic acid, linolic acid, α-linoleic acid and palmitic acid.

The composition of the test diet was 28.5% of starch, 25% of TG, 5% of lard, 13% of sucrose, 20% of casein, 3.5% of AIN76 mineral mixture, and 1% of AIN76 vitamin mixture.

Seven-week old male mice C57BL/6J Jcl (CREA Japan, Inc.) were bred on an ordinary diet (CRF-1: Oriental Yeast Co., Ltd.) for one week, then the mice were grouped such that the initial body weights of the mice were nearly uniform at the time of 8 weeks old, and the test was initiated. Breeding of the mice was performed with five animals per cage, and two cages (N = 10) were assigned for each of the test diet groups. Feeding was performed by free feeding using a Roden Café (Oriental Yeast Co., Ltd.), and fresh test diet was replaced every 2 or 3 days. The test diet used was divided in advance into portions for 2 to 3 days, and stored under refrigeration at 4°C until the time of use. Water feeding was achieved by freely feeding tap water using a waterer for exclusive use. The amount of food ingestion and the body weight were measured every week during the test breeding period.

### (3) Analysis of relevance between postprandial insulin secretion and obesity

An analysis was performed on the correlation between the maximum blood sugar level and the maximum blood insulin level after ingesting carbohydrate only, or both carbohydrate and lipid, and the increase in the body weight during the breeding period.

The results are presented in Table 3. The increase in body weight (g) per gram of the amount of ingestion during a breeding period of 10 weeks, was proved to have high positive correlation with the increase in the blood sugar level, and with the insulin secretion after intake of both carbohydrate (test starch) and lipid, compared to the case of the insulin secretion after intake of carbohydrate (test starch) only. Thus, it was found that the insulin secretion after intake of both carbohydrate and lipid is a factor highly correlated with obesity.

**[Table 3]**

| Correlation coefficient for body weight increase | | | | |
|---|---|---|---|---|
| | Carbohydrate (test starch) alone | | Carbohydrate (test starch) + lipid | |
| | Maximum blood sugar level | Maximum insulin level | Maximum blood sugar level | Maximum insulin level |
| Week 1 | -0.27 | -0.08 | 0.08 | 0.09 |
| Week 2 | -0.37 | 0.01 | 0.20 | 0.36 |
| Week 3 | -0.66 | 0.32 | 0.03 | 0.44 |
| Week 4 | -0.51 | 0.51 | 0.16 | 0.69 |
| Week 5 | -0.32 | 0.57 | 0.25 | 0.74 |
| Week 6 | -0.36 | 0.59 | 0.44 | 0.76 |
| Week 7 | -0.22 | 0.40 | 0.31 | 0.63 |
| Week 8 | -0.16 | 0.45 | 0.60 | 0.76 |
| Week 9 | 0.04 | 0.46 | 0.58 | 0.78 |
| Week 10 | 0.15 | 0.42 | 0.61 | 0.72 |

### EXAMPLE 3: Low insulin secretion inducibility of glycogen

Triolein (Sigma Corp.) was used as the lipid, while glucose (Wako Pure Chemical Industries, Ltd.) or glycogen (derived from sweet corn, QP Corp.) was used as the carbohydrate.

Nine to ten fasted mice (C57BL/6J, male, 10 to 11 weeks old) were grouped in each group, and the mice were orally administered, using a sonde, with 2 mg/g of bodyweight of glucose; with the glucose and also 2 mg/g of body weight of triolein, in the form of an emulsion obtained by emulsifying 0.02 mg/g of body weight of the lipid with egg yolk lecithin (glucose, and glucose + lipid, respectively) ; with 2 mg/g of body weight of glycogen; or with the glycogen and also 2 mg/g of body weight of triolein (TAG), in the form of an emulsion obtained by emulsifying 0.02 mg/g of body weight of the lipid with egg yolk lecithin (glycogen, and glycogen + lipid, respectively). The compositions of the administered products are presented in Table 4. The blood was sampled from the orbital vein of each mouse before the administration, and at 10 and 30 minutes after administration, the blood insulin level was measured, and the area under curve (AUC) of the graph was calculated. The insulin level was measured by the ELISA method (insulin measuring kit, Morinaga Biochemical Lab, Inc.).

**[Table 4]**

| | Glucose (mg/g of body weight) | Glycogen (mg/g of body weight) | Triolein (mg/g of body weight) |
|---|---|---|---|
| Glucose | 2.0 | 0.0 | 0.0 |
| Glycogen | 0.0 | 2.0 | 0.0 |
| Glucose + lipid | 2.0 | 0.0 | 2.0 |
| Glycogen + lipid | 0.0 | 2.0 | 2.0 |

The values of the amount of secreted insulin (AUC) for a period of 30 minutes after the administration are presented in Table 5.

**[Table 5]**

| Amount of postprandial insulin secretion in mouse (AUC) | |
|---|---|
| | Amount of postprandial insulin secretion (average) |
| Glucose | 38.8 |
| Glycogen | 33.4 |
| Glucose + lipid | 124.5 |
| Glycogen + lipid | 77.0 |

From the results of Table 5, the postprandial insulin secretion was equivalent in the case of ingesting glucose only or glycogen only, but when ingested together with lipid, glycogen resulted in a lower amount of postprandial insulin secretion, compared to glucose. Thus, it can be seen that glycogen has low insulin secretion inducibility in the co-presence of lipid.

### EXAMPLE 4: Anti-obesity effect of glycogen

### (1) Test diet

The composition of the test diet (powdered diet) is presented in Table 6. The low fat diet contained 5% of lipid (triglyceride), and the high fat diet contained 30% of lipid (5% lard + 25% TG).

The glycogen mix diet was prepared by incorporating 5%, 10% or 28.5% of glycogen to the high fat diet. The glycogen used in the test diet was glycogen derived from sweet corn (QP Corp.), while lard, casein, cellulose, AIN76 mineral mixture, AIN76 vitamin mixture and gelatinized potato starch were obtained from Oriental Yeast Co., Ltd. , and sucrose fine granules (Special Grade) manufactured by Wako Pure Chemical Industries, Ltd. were used as sucrose. Also, the oil and fat (TG) used was a mixture of high linoleic safflower oil, rapeseed oil and perilla oil, and the main components of fatty acid were oleic acid, linolic acid, α-linoleic acid and palmitic acid.

**[Table 6]**

| Table of test diet composition | | | | | |
|---|---|---|---|---|---|
| Incorporated (%) | Low fat diet | High fat diet | | | |
| | | Proportion of incorporated glycogen | | | |
| | | Control (0%) | 5% | 10% | 28.5% |
| TG | 5 | 25 | 25 | 25 | 25 |
| Lard | - | 5 | 5 | 5 | 5 |
| Glycogen | - | 0 | 5 | 10 | 28.5 |
| Sucrose | - | 13 | 13 | 13 | 13 |
| Casein | 20 | 20 | 20 | 20 | 20 |
| Cellulose | 4 | 4 | 4 | 4 | 4 |
| AIN76 mineral mixture | 3.5 | 3.5 | 3.5 | 3.5 | 3.5 |
| AIN76 vitamin mixture | 1 | 1 | 1 | 1 | 1 |
| Gelatinized potato starch | 66.5 | 28.5 | 23.5 | 18.5 | 0 |
| Total (%) | 100 | 100 | 100 | 100 | 100 |

### (2) Test animal and breeding thereof

Seven-week old male mice C57BL/6J Jcl (CREA Japan, Inc.) were bred on an ordinary diet (CRF-1: Oriental Yeast Co., Ltd.) for one week, then the mice were grouped such that the initial body weights of the mice were nearly uniform at the time of 8 weeks old, and the test was initiated. Breeding of the mice was performed with five animals per cage, and two cages (N = 10) were assigned for each of the test diet groups. Feeding was performed by free feeding using a Roden Café (Oriental Yeast Co. , Ltd.), and fresh test diet was replaced every 2 or 3 days. The test diet used was divided in advance into portions for 2 to 3 days, and stored under refrigeration at 4°C until the time of use. Water feeding was achieved by freely feeding tap water using a waterer for exclusive use.

### (3) Body weight measurement and collection of visceral fat

The body weight was measured every week during the test breeding period. On the last day of experiment, the mice were freely fed until immediately before the dissection, and thus the visceral fat was collected under non-fasting conditions, as disclosed below. A mouse was immediately subjected to laparotomy under anesthesia, the blood was collected from the abdominal aorta, and the mouse was left to bleed to death. More visceral fat (fat around epididymidis, fat around kidneys, retroperitoneal fat, and mesenteric fat) was collected, the weight was measured, and the total value was calculated and taken as the amount of visceral fat.

### (4) Results

Since the mice were subjected to collective breeding with 5 animals per cage, there were fights among the mice during a breeding period of 15 weeks. Thus, two animals which were recognized to have sudden and rapid weight loss were excluded.

The results are presented in Table 7. It can be seen that the high fat diet (control) caused increases in the body weight and the amount of visceral fat during a 15-week period of breeding, as compared to the low fat diet, thus leading to obesity.

As the amount of glycogen incorporated increased, the body weight and the amount of visceral fat decreased.

**[Table 7]**

| Body weight and amount of visceral fat (average) of mouse after breeding of 15 weeks | | | | | |
|---|---|---|---|---|---|
| | Low fat diet | High fat diet | | | |
| | | Amount of incorporated glycogen (%) | | | |
| | | Control (0%) | 5% | 10% | 28.5% |
| Body weight (g) | 33.7 | 36.6 | 36.4 | 34.4 | 32.0 |
| Amount of visceral fat (g) | 2.02 | 2.88 | 2.83 | 2.43 | 1.77 |

### EXAMPLE 5: Effect of reducing insulin secretion by 1-MAG

Triolein (obtained from Sigma Corp.) was used as triacylglycerol, and 1-monoolein (obtained from Sigma Corp.) was used as monoacylglycerol. Glucose (Kanto Chemical Co., Inc.) was used as the carbohydrate.

Eight fasted mice (C57BL/6J, male, 8 weeks old) were grouped in each group, and the mice were orally administered, using a sonde, with 2 mg/g of body weight of glucose only; with the glucose and also 2 mg/g of body weight of triolein (TAG), in the form of an emulsion obtained by emulsifying 0.02 mg/g of body weight of the lipid with egg yolk lecithin (Glucose and TAG/MAG0, respectively); or with mixtures obtained by respectively adding 0.08, 0.2 and 0.4 mg/g of body weight of 1-monoolein (MAG) to the emulsion (TAG/MAG1, TAG/MAG2, and TAG/MAG3, respectively). The compositions of the emulsions are presented in Table 8. The blood was sampled from the orbital vein of each mouse before the administration, and at 10 and 30 minutes after the administration, the blood insulin level was measured, and the area under curve (AUC) of the graph was calculated. The insulin level was measured by the ELISA method (insulin measuring kit, Morinaga Biochemical Lab, Inc.).

Relative values of the amount of secreted insulin (AUC) for 30 minutes after the administration, with respective to the insulin AUC value of the mice which ingested glucose only, taken as 100, are presented in Table 9.

**[Table 8]**

| Emulsion | Glucose (mg/g of body weight) | Triolein (mg/g of body weight) | 1-Monoolein (mg/g of body weight) |
|---|---|---|---|
| Glucose | 2.0 | 0.0 | 0.0 |
| TAG/MAG0 | 2.0 | 2.0 | 0.0 |
| TAG/MAG1 | 2.0 | 2.0 | 0.08 |
| TAG/MAG2 | 2.0 | 2.0 | 0.2 |
| TAG/MAG3 | 2.0 | 2.0 | 0.4 |

**[Table 9]**

| Amount of postprandial insulin secretion in mouse (AUC, relative value) | |
|---|---|
| Emulsion composition | Amount of postprandial insulin secretion (average) |
| Glucose | 100 |
| TAG/MAG0 | 166.7 |
| TAG/MAG1 | 147.0 |
| TAG/MAG2 | 115.0 |
| TAG/MAG3 | 86.0 |

From the results of Table 9, it can be seen that the amount of postprandial insulin secretion increases, when lipid (TAG) is ingested, as compared to the case of ingesting carbohydrate (glucose) only, but the mice which ingested MAG in a one-tenth amount or a one-fifth amount relative to the amount of TAG/MAG0, showed low amounts of postprandial insulin secretion, and an effect of suppressing the postprandial insulin secretion in the blood was recognized.

### EXAMPLE 6: Anti-obesity effect of 1-MAG

### (1) Test diet

The composition of the test diet (powdered diet) is presented in Table 10. The low fat diet contained 5% of lipid (TG), and the high fat diet contained 30% of lipid (TG).

The 1-monoacylglyceride (1-MAG) mix diet was prepared by incorporating 3% or 6% of 1-MAG to the high fat diet.

The 1-MAG used in the test diet was Excel O-95R (KaoCorp.), while casein, cellulose, AIN76 mineral mixture, AIN76 vitamin mixture andgelatinizedpotato starch were obtained from Oriental Yeast Co., Ltd., and sucrose fine granules (Special Grade) manufactured by Wako Pure Chemical Industries, Ltd. were used as sucrose. Also, the oil and fat (TG) used was a mixture of high linoleic safflower oil, rapeseed oil and perilla oil, and the main components of fatty acid were oleic acid, linolic acid, α-linoleic acid and palmitic acid.

**[Table 10]**

| Table of test diet composition | | | | |
|---|---|---|---|---|
| Incorporated (%) | Low fat diet | High fat diet | | |
| | | Proportion of incorporated 1-MAG | | |
| | | Control (0%) | 3% | 6% |
| TG | 5 | 30 | 30 | 30 |
| 1-MAG | - | 0 | 3 | 6 |
| Sucrose | - | 13 | 13 | 13 |
| Casein | 20 | 20 | 20 | 20 |
| Cellulose | 4 | 4 | 4 | 4 |
| AIN76 mineral mixture | 3.5 | 3.5 | 3.5 | 3.5 |
| AIN76 vitamin mixture | 1 | 1 | 1 | 1 |
| Gelatinized potato starch | 66.5 | 28.5 | 25.5 | 22.5 |
| Total (%) | 100 | 100 | 100 | 100 |

### (1) Test animal and breeding thereof

Seven-week old male mice C57BL/6J Jcl (CREA Japan, Inc.) were bred on an ordinary diet (CE-2: CREA Japan, Inc.) for one week, then the mice were grouped such that the initial bodyweights of the mice were nearly uniform at the time of 8 weeks old, and the test was initiated. Breeding of the mice was performed with four animals per cage, and two cages (N = 8) were assigned for each of the test diet groups. Feeding was performed by free feeding using a Roden Café (Oriental Yeast Co. , Ltd.), and fresh test diet was replaced every 2 or 3 days. The test diet used was divided in advance into portions for 2 to 3 days, and stored under refrigeration at 4°C until the time of use. Water feeding was achieved by freely feeding tap water using a waterer for exclusive use.

### (2) Body weight measurement and collection of visceral fat

The body weight was measured every week during the test breeding period. On the last day of experiment, the mice were freely fed until immediately before the dissection, and thus the visceral fat was collected under non-fasting conditions, as disclosed below. A mouse was immediately subjected to laparotomy under anesthesia, the blood was collected from the abdominal aorta, and the mouse was left to bleed to death. More visceral fat (fat around epididymidis, fat around kidneys, retroperitoneal fat, and mesenteric fat) was collected, the weight was measured, and the total value was calculated and taken as the amount of visceral fat.

### (3) Results

The results are presented in Table 11. It can be seen that the high fat diet (control) caused increases in the body weight and the amount of visceral fat during a 9-week period of breeding, as compared to the low fat diet, thus leading to obesity.

As the amount of 1-MAG incorporated increased, the body weight and the amount of visceral fat decreased.

**[Table 11]**

| Body weight and amount of visceral fat of mouse after breeding of 9 weeks | | | | |
|---|---|---|---|---|
| | Low fat diet | High fat diet | | |
| | | Amount of incorporated 1-MAG (%) | | |
| | | Control (0%) | 3% | 6% |
| Body weight (g) | 29.3 | 32.9 | 31.2 | 28.3 |
| Amount of visceral fat (g) | 1.56 | 2.46 | 2.05 | 1.50 |

### EXAMPLE 7: Low insulin secretion inducibility of hydroxypropylated phosphate crosslinked starch

Processed starches are known to have an obesity ameliorating effect, in contrast to high amylose starches (JP-A -2004-269458). Insulin secretion upon ingesting processed starch together with lipid was compared with insulin secretion upon ingesting high amylose starch.

Hydroxypropylated phosphate crosslinked starch (hereinafter, processed starch) (derived from tapioca, National Freejeks, obtained from National Starch and Chemical Company) and high amylose starch (derived from high amylose corn, Fibos, obtained from Nippon Starch Chemical Co., Ltd.) were used as the carbohydrate, and triolein (Sigma Corp.) was used as the lipid.

Eight fasted mice (C57BL/6J, male, 8 weeks old) were grouped in each group, and the mice were orally administered, using a sonde, with 2 mg/g of body weight of processed starch or high amylose starch, or with the carbohydrate and also 2 mg/g of body weight of lipid, in the form of an emulsion obtained by emulsifying 0.02 mg/g of body weight of the fat with egg yolk lecithin (processed starch + lipid, and high amylose starch + lipid, respectively). The blood was sampled from the orbital vein of each mouse before administration, and at 10, 30 and 60 minutes after the administration, the blood insulin level was measured, and the area under curve (AUC) of the graph was calculated. Measurement of the insulin level was performed by the ELISA method (insulin measuring kit, Morinaga Biochemical Lab, Inc.).

The values of the amount of secreted insulin (AUC) for a period of 60 minutes after administration are presented in Table 12.

**[Table 12]**

| Amount of postprandial insulin secretion of mouse | |
|---|---|
| | Amount of postprandial insulin secretion (average AUC) |
| Processed starch + lipid | 55.1 |
| High amylose starch + lipid | 125 |

From the results of Table 12, it can be seen that when ingested together with lipid, the processed starch results in a low amount of postprandial insulin secretion, compared to the high amylose starch, and the processed starch has low insulin secretion inducibility in the co-presence of lipid, as compared to the high amylose starch.

### EXAMPLE 8: Low insulin secretion inducibility of DAG and fish oil

The insulin secretion of when diacylglycerol, which is a lipid known to have an obesity ameliorating effect, and fish oil were ingested together with carbohydrate, was compared with the insulin secretion of triacylglycerol.

Triacylglycerol (TAG), diacylglycerol (DAG, Kao Corp.) and fish oil (obtained from NOF Corp., DHA content 47%) were used as the lipids. Also, the oil and fat (TAG) used was a mixture of high linoleic safflower oil, rapeseed oil and perilla oil, and the main components of the fatty acid were oleic acid, linolic acid, α-linoleic acid and palmitic acid, being the same as DAG.

Ten fasted mice (C57BL/6J, male, 8 weeks old) were grouped in each group, and the mice were orally administered, using a sonde, with 2 mg/g of body weight of glucose only; or with the carbohydrate and also 2 mg/g of body weight of triacylglycerol (TAG), diacylglycerol (DAG) or fishoil, in the form of an emulsion obtained by emulsifying 0.02 mg/g of body weight of the lipid with egg yolk lecithin (carbohydrate only, carbohydrate + TAG, carbohydrate + DAG, and carbohydrate + fish oil, respectively). The blood was sampled from the orbital vein before the administration, and at 10 and 30 minutes after the administration, the blood insulin level was measured, and the area under curve (AUC) of the graph was calculated. Measurement of the insulin level was performed by the ELISA method (insulin measuring kit, Morinaga Biochemical Lab, Inc.).
The relative values of the amount of secreted insulin for a period of 30 minutes after the administration, with respect to the insulin AUC of a mouse which ingested glucose only, taken as 100, are presented in Table 13.

**[Table 13]**

| Amount of postprandial insulin secretion of mouse (AUC, relative values) | |
|---|---|
| | Amount of postprandial insulin secretion (average) |
| Carbohydrate only | 100 |
| Carbohydrate + TAG | 236.4 |
| Carbohydrate + DAG | 140.2 |
| Carbohydrate + fish oil | 155.8 |

From the results of Table 13, it can be seen that when triacylglycerol is ingested as the lipid, the amount of postprandial insulin secretion increases, as compared to the case of ingesting carbohydrate (glucose) only, but a mouse which ingested diacylglycerol or fish oil as the lipid exhibits a smaller amount of postprandial insulin secretion compared to the case of carbohydrate + TAG, and an effect of suppressing postprandial insulin secretion in the blood is recognized.

DAG (Murase T, et al., J. Lipid Res. 2001, 42:372-378) and fish oil (Ikemoto S, et al. , Metabolism 1996, 45: 1539-1546) are known to have an obesity ameliorating effect as compared with common lipids.

## Claims

1. A method for evaluating or screening an obesity controller, the method comprising administering a test substance together with a carbohydrate and lipid to an animal, measuring the blood insulin level by sampling the blood before administration and at time points between 1 to 300 minutes after the administration time, measuring the blood insulin level at each time point and determining an amount of secreted insulin and evaluating or selecting the substance which decreases or increases insulin secretion.

2. A method for evaluating or screening an obesity controller, the method comprising the following processes (1) to (4):
(1) a process of administering a test substance to an animal by the following methods a) and/or b):
a) the test substance is added to a carbohydrate and a lipid, and/or
b) a part or all of at least one of the carbohydrate and the lipid is replaced with the test substance;
(2) a process of measuring a blood insulin level by sampling the blood before administration and at time points between 1 to 300 minutes after the administration time, measuring a blood insulin level at each time point and determining an amount of secreted insulin;
(3) a process of comparing the amount of the secreted insulin determined in Process (2), with an amount of insulin secreted in a control group to which only the carbohydrate and the lipid have been administered; and
(4) a process of evaluating or selecting, on the basis of the results of Process (3), the test substance which decreases or increases insulin secretion, as the obesity controller.

3. A method for evaluating or screening a blood insulin regulator, the method comprising administering a test substance together with a carbohydrate and a lipid to an animal, measuring the blood insulin level by sampling the blood before administration and at time points between 1 to 300 minutes after the administration time, measuring the blood insulin level at each time point and determining an amount of secreted insulin and evaluating or selecting the substance which decreases or increases insulin secretion.

4. A method for evaluating or screening a blood insulin regulator, the method comprising the following processes (1) to (4):
(1) a process of administering a test substance to an animal by the following methods a) and/or b):
a) the test substance is added to a carbohydrate and a lipid, and/or
b) a part or all of at least one of the carbohydrate and the lipid is replaced with the test substance;
(2) a process of measuring a blood insulin level by sampling the blood before administration and at time points between 1 to 300 minutes after the administration time, measuring a blood insulin level at each time point, and determining an amount of secreted insulin;
(3) a process of comparing the amount of the secreted insulin determined in Process (2), with an amount of insulin secreted in a control group to which only the carbohydrate and the lipid have been administered; and
(4) a process of evaluating or selecting, on the basis of the results of Process (3), the test substance which decreases or increases insulin secretion, as the blood insulin regulator.

5. A method for evaluating or screening a blood sugar regulator, the method comprising administering a test substance together with a carbohydrate and a lipid to an animal, measuring the blood insulin level by sampling the blood before administration and at time points between 1 to 300 minutes after the administration time, measuring the blood insulin level at each time point and determining an amount of secreted insulin and evaluating or selecting the substance which decreases or increases insulin secretion.

6. A method for evaluating or screening a blood sugar regulator, the method comprising the following processes (1) to (4):
(1) a process of administering a test substance to an animal by the following methods a) and/or b):
a) the test substance is added to a carbohydrate and a lipid, and/or
b) a part or all of at least one of the carbohydrate and the lipid is replaced with the test substance;
(2) a process of measuring a blood insulin level by sampling the blood before administration and at time points between 1 to 300 minutes after the administration time, measuring a blood insulin level at each time point, and determining an amount of secreted insulin;
(3) a process of comparing the amount of the secreted insulin determined in Process (2), with the amount of insulin secreted in a control group to which only the carbohydrate and the lipid have been administered; and
(4) a process of evaluating or selecting, on the basis of the results of Process (3), the test substance which decreases or increases insulin secretion, as the blood sugar regulator.

7. The method according to any one of claims 1 to 6, wherein the carbohydrate is at least one selected from glucose, sucrose, maltose, starch and glycogen, and the lipid is at least one selected from triacylglycerol, 1,3-diacylglycerol and 1,2-diacylglycerol.

8. The method according to any one of claims 1 to 7, wherein the animal is a rodent.

## Patentansprüche

1. Verfahren zum Evaluieren oder Screenen eines Fettsuchtreglers, umfassend das Verabreichen einer Testsubstanz zusammen mit einem Kohlenhydrat und Lipid an ein Tier, das Messen des Blutinsulinspiegels durch Probeentnahme des Blutes vor der Verabreichung und zu Zeitpunkten zwischen 1 bis 300 Minuten nach der Verabreichungszeit, das Messen des Blutinsulinspiegels zu jedem Zeitpunkt und das Bestimmen einer Menge an sekretiertem Insulin und das Evaluieren oder Selektieren der Substanz, die die Insulinsekretion herabsetzt oder erhöht.

2. Verfahren zum Evaluieren oder Screenen eines Fettsuchtreglers, wobei das Verfahren die folgenden Prozesse (1) bis (4) umfasst:
(1) Prozess des Verabreichens einer Testsubstanz an ein Tier durch die folgenden Verfahren a) und/oder b):
a) die Testsubstanz wird einem Kohlenhydrat und einem Lipid zugefügt, und/oder
b) ein Teil oder alles von mindestens dem Kohlenhydrat und dem Lipid wird mit der Testsubstanz ersetzt;
(2) Prozess des Messens des Blutinsulinspiegels durch Probeentnahme des Blutes vor der Verabreichung und zu Zeitpunkten zwischen 1 bis 300 Minuten nach der Verabreichungszeit, des Messens eines Blutinsulinspiegels zu jedem Zeitpunkt und des Bestimmens einer Menge an sekretiertem Insulin;
(3) Prozess des Vergleichens der Menge an sekretiertem Insulin, die in Prozess (2) bestimmt wurde, mit der Menge an sekretiertem Insulin in einer Kontrollgruppe, der nur das Kohlenhydrat und das Lipid verabreicht wurde; und
(4) Prozess des Evaluierens oder Selektierens, auf der Basis der Ergebnisse von Prozess (3), der Testsubstanz, welche die Insulinsekretion herabsetzt oder erhöht, als der Fettsuchtregler.

3. Verfahren zum Evaluieren oder Screenen eines Blutinsulinregulators, umfassend das Verabreichen einer Testsubstanz zusammen mit einem Kohlenhydrat und einem Lipid an ein Tier, das Messen des Blutinsulinspiegels durch Probeentnahme des Blutes vor der Verabreichung und zu Zeitpunkten zwischen 1 bis 300 Minuten nach der Verabreichungszeit, das Messen des Blutinsulinspiegels zu jedem Zeitpunkt und das Bestimmen einer Menge an sekretiertem Insulin und das Evaluieren oder Selektieren der Substanz, die die Insulinsekretion herabsetzt oder erhöht.

4. Verfahren zum Evaluieren oder Screenen eines Blutinsulinreglers, wobei das Verfahren die folgenden Prozesse (1) bis (4) umfasst:
(1) Prozess des Verabreichens einer Testsubstanz an ein Tier durch die folgenden Verfahren a) und/oder b):
a) die Testsubstanz wird einem Kohlenhydrat und einem Lipid zugefügt, und/oder
b) ein Teil oder alles von mindestens dem Kohlenhydrat und dem Lipid wird mit der Testsubstanz ersetzt;
(2) Prozess des Messens des Blutinsulinspiegels durch Probeentnahme des Blutes vor der Verabreichung und zu Zeitpunkten zwischen 1 bis 300 Minuten nach der Verabreichungszeit, des Messens eines Blutinsulinspiegels zu jedem Zeitpunkt und des Bestimmens einer Menge an sekretiertem Insulin;
(3) Prozess des Vergleichens der Menge an sekretiertem Insulin, die in Prozess (2) bestimmt wurde, mit der Menge an sekretiertem Insulin in einer Kontrollgruppe, der nur das Kohlenhydrat und das Lipid verabreicht wurde; und
(4) Prozess des Evaluierens oder Selektierens, auf der Basis der Ergebnisse von Prozess (3), der Testsubstanz, welche die Insulinsekretion herabsetzt oder erhöht, als der Blutinsulinregulator.

5. Verfahren zum Evaluieren oder Screenen eines Blutzuckerregulators, umfassend das Verabreichen einer Testsubstanz zusammen mit einem Kohlenhydrat und einem Lipid an ein Tier, das Messen des Blutinsulinspiegels durch Probeentnahme des Blutes vor der Verabreichung und zu Zeitpunkten zwischen 1 bis 300 Minuten nach der Verabreichungszeit, das Messen des Blutinsulinspiegels zu jedem Zeitpunkt und das Bestimmen einer Menge an sekretiertem Insulin und das Evaluieren oder Selektieren der Substanz, die die Insulinsekretion herabsetzt oder erhöht.

6. Verfahren zum Evaluieren oder Screenen eines Blutzuckerregulators, wobei das Verfahren die folgenden Prozesse (1) bis (4) umfasst:
(1) Prozess des Verabreichens einer Testsubstanz an ein Tier durch die folgenden Verfahren a) und/oder b):
a) die Testsubstanz wird einem Kohlenhydrat und einem Lipid zugefügt, und/oder
b) ein Teil oder alles von mindestens dem Kohlenhydrat und dem Lipid wird mit der Testsubstanz ersetzt;
(2) Prozess des Messens des Blutinsulinspiegels durch Probeentnahme des Blutes vor der Verabreichung und zu Zeitpunkten zwischen 1 bis 300 Minuten nach der Verabreichungszeit, des Messens eines Blutinsulinspiegels zu jedem Zeitpunkt und des Bestimmens einer Menge an sekretiertem Insulin;
(3) Prozess des Vergleichens der Menge an sekretiertem Insulin, die in Prozess (2) bestimmt wurde, mit der Menge an sekretiertem Insulin in einer Kontrollgruppe, der nur das Kohlenhydrat und das Lipid verabreicht wurde; und
(4) Prozess des Evaluierens oder Selektierens, auf der Basis der Ergebnisse von Prozess (3), der Testsubstanz, welche die Insulinsekretion herabsetzt oder erhöht, als der Blutzuckerregulator.

7. Verfahren gemäß einem der Ansprüche 1 bis 6, wobei das Kohlenhydrat mindestens eins ist, ausgewählt aus Glukose, Saccharose, Maltose, Stärke und Glycogen und das Lipid mindestens eins ist, ausgewählt aus Triacylglycerol, 1,3-Diacylglycerol und 1,2-Diacylglycerol.

8. Verfahren gemäß einem der Ansprüche 1 bis 7, wobei das Tier ein Nagetier ist.

## Revendications

1. Procédé d'évaluation ou de criblage d'un contrôleur de l'obésité, le procédé comprenant l'administration d'une substance à tester conjointement avec un glucide et un lipide à un animal, la mesure du taux d'insuline dans le sang par échantillonnage du sang avant l'administration et à des points du temps entre 1 et 300 minutes après le moment de l'administration, la mesure du taux d'insuline dans le sang à chaque point du temps et la détermination d'une quantité d'insuline sécrétée et l'évaluation ou la sélection de la substance qui diminue ou augmente la sécrétion d'insuline.

2. Procédé d'évaluation ou de criblage d'un contrôleur de l'obésité, le procédé comprenant les procédés (1) à (4) suivants :
(1) un procédé d'administration d'une substance à tester à un animal par les procédés a) et/ou b) suivants :
a) la substance à tester est ajoutée à un glucide et un lipide, et/ou
b) une partie ou la totalité d'au moins l'un du glucide et du lipide est remplacée par la substance à tester ;
(2) un procédé de mesure d'un taux d'insuline dans le sang par échantillonnage du sang avant l'administration et à des points du temps entre 1 et 300 minutes après le moment de l'administration, la mesure d'un taux d'insuline dans le sang à chaque point du temps et la détermination d'une quantité d'insuline sécrétée ;
(3) un procédé de comparaison de la quantité d'insuline sécrétée déterminée dans le procédé (2), avec une quantité d'insuline sécrétée dans un groupe contrôle auquel seuls le glucide et le lipide ont été administrés ; et
(4) un procédé d'évaluation ou de sélection, sur la base des résultats du procédé (3), de la substance à tester qui diminue ou augmente la sécrétion d'insuline, en tant que contrôleur de l'obésité.

3. Procédé d'évaluation ou de criblage d'un régulateur de l'insuline dans le sang, le procédé comprenant l'administration d'une substance à tester conjointement avec un glucide et un lipide à un animal, la mesure du taux d'insuline dans le sang par échantillonnage du sang avant l'administration et à des points du temps entre 1 et 300 minutes après le moment de l'administration, la mesure du taux d'insuline dans le sang à chaque point du temps et la détermination d'une quantité d'insuline sécrétée et l'évaluation ou la sélection de la substance qui diminue ou augmente la sécrétion d'insuline.

4. Procédé d'évaluation ou de criblage d'un régulateur de l'insuline dans le sang, le procédé comprenant les procédés (1) à (4) suivants :
(1) un procédé d'administration d'une substance à tester à un animal par les procédés a) et/ou b) suivants :
a) la substance à tester est ajoutée à un glucide et un lipide, et/ou
b) une partie ou la totalité d'au moins l'un du glucide et du lipide est remplacée par la substance à tester ;
(2) un procédé de mesure d'un taux d'insuline dans le sang par échantillonnage du sang avant l'administration et à des points du temps entre 1 et 300 minutes après le moment de l'administration, la mesure d'un taux d'insuline dans le sang à chaque point du temps, et la détermination d'une quantité d'insuline sécrétée ;
(3) un procédé de comparaison de la quantité d'insuline sécrétée déterminée dans le procédé (2), avec une quantité d'insuline sécrétée dans un groupe contrôle auquel seuls le glucide et le lipide ont été administrés ; et
(4) un procédé d'évaluation ou de sélection, sur la base des résultats du procédé (3), de la substance à tester qui diminue ou augmente la sécrétion d'insuline, en tant que régulateur de l'insuline dans le sang.

5. Procédé d'évaluation ou de criblage d'un régulateur du sucre dans le sang, le procédé comprenant l'administration d'une substance à tester conjointement avec un glucide et un lipide à un animal, la mesure du taux d'insuline dans le sang par échantillonnage du sang avant l'administration et à des points du temps entre 1 et 300 minutes après le moment de l'administration, la mesure du taux d'insuline dans le sang à chaque point du temps et la détermination d'une quantité d'insuline sécrétée et l'évaluation ou la sélection de la substance qui diminue ou augmente la sécrétion d'insuline.

6. Procédé d'évaluation ou de criblage d'un régulateur du sucre dans le sang, le procédé comprenant les procédés (1) à (4) suivants :
(1) un procédé d'administration d'une substance à tester à un animal par les procédés a) et/ou b) suivants :
a) la substance à tester est ajoutée à un glucide et un lipide, et/ou
b) une partie ou la totalité d'au moins l'un du glucide et du lipide est remplacée par la substance à tester ;
(2) un procédé de mesure d'un taux d'insuline dans le sang par échantillonnage du sang avant l'administration et à des points du temps entre 1 et 300 minutes après le moment de l'administration, la mesure d'un taux d'insuline dans le sang à chaque point du temps, et la détermination d'une quantité d'insuline sécrétée ;
(3) un procédé de comparaison de la quantité d'insuline sécrétée déterminée dans le procédé (2), avec une quantité d'insuline sécrétée dans un groupe contrôle auquel seuls le glucide et le lipide ont été administrés ; et
(4) un procédé d'évaluation ou de sélection, sur la base des résultats du procédé (3), de la substance à tester qui diminue ou augmente la sécrétion d'insuline, en tant que régulateur du sucre dans le sang.

7. Procédé selon l'une quelconque des revendications 1 à 6, dans lequel le glucide est au moins l'un choisi parmi le glucose, le saccharose, le maltose, l'amidon et le glycogène, et le lipide est au moins l'un choisi parmi le triacylglycérol, le 1,3-diacylglycérol et le 1,2-diacylglycérol.

8. Procédé selon l'une quelconque des revendications 1 à 7, dans lequel l'animal est un rongeur.
